# EUROPEAN PATENT SPECIFICATION

(11) **EP 3 003 234 B1**
(45) Date of publication and mention of the grant of the patent: **30.06.2021**
(21) Application number: 14807041.0
(22) Date of filing: 26.05.2014
(51) Int. Cl.: A61F 5/56, A61C 7/08

(54) **INCREMENTAL ADJUSTABLE MANDIBULAR ADVANCEMENT DEVICE FOR PREVENTING AND TREATMENT OF SNORING AND OBSTRUCTIVE SLEEP APNEA**
SCHRITTWEISE EINSTELLBARE MANDIBULÄRE VORSCHUBVORRICHTUNG ZUR VORBEUGUNG UND BEHANDLUNG VON SCHNARCHEN UND OBSTRUKTIVER SCHLAFAPNOE
DISPOSITIF D'AVANCEMENT MANDIBULAIRE RÉGLABLE DE MANIÈRE INCRÉMENTALE ET DESTINÉ À LA PRÉVENTION ET AU TRAITEMENT DES RONFLEMENTS ET DE L'APNÉE DU SOMMEIL OBSTRUCTIVE

(30) Priority: 02.06.2013 DK 201300338
(43) Date of publication of application: 13.04.2016
(73) Proprietor: Ingemarsson-Matzen, Natashia, 2920 Charlottenlund (DK)
(72) Inventor: Ingemarsson-Matzen, Natashia, 2920 Charlottenlund (DK)
(74) Representative: Schmidt, Johnny Olesen
(86) International application number: PCT/DK2014/000030
(87) International publication number: WO 2014/194910

(56) References cited:
- WO-A2-2013/049751
- DE-U1- 20 102 432
- DE-U1-202008 010 330
- US-A- 6 161 542
- US-A1- 2012 199 136
- US-B1- 6 729 335
- US-B1- 7 520 281
- US-B1- 7 810 502

## Description

### FIELD OF INVENTION

The current invention relates to an adjustable mandibular advancement device which by virtue of an incremental (stepwise) mechanism, advances or withdraws the mandibular relative to the maxilla in order to prevent or reduce Snoring and/or Obstructive Sleep Apnea Syndrome (OSAS) during sleep. The adjustability is accomplished by the intrinsic embedded mechanism in the two members in conjunction or separately in either of the members relative to the other. Document WO 2013/049751 A2 is regarded as the closest prior art.

### BACKGROUND OF THE INVENTION

Snoring and Obstructive Sleep Apnea are generally known today as the same disease on a continuum of the sleep disorder severity scale. Starting at the modest degree of snoring ending in the fulminate obstructive sleep apnea condition, is known as a fact. As the disease is closely related to a large variety of physical and mental conditions, treatment is of outmost importance as soon as possible.

Whereas the snoring condition is characterized by the sounds developed by vibrating tissues in the most dorsal area of the pharynx, either the nasopharynx, or the oropharynx or the laryngopharynx, the obstructive sleep apnea is characterized by actual respiration arrest caused by occlusion of the pharyngeal airways.

Apnea appears when the upper airway passages are being sucked close to the rear part of the throat when the person is trying to breathe during sleep. The occlusion can be the result of suction or by the lapse of tonus in the oral soft tissues during the relaxed sleep condition.

When the occlusion is there, no air is passing through the pharynx and down to the lungs, and this is the situation called OSAS (Obstructive Sleep Apnea Syndrome).

The obstruction can happen as often as 1000 times during the night time sleep in which the body is depraved from oxygen uptake from the air into the blood stream, which eventually leads to the aggravated symptoms.

The severity of OSAS has been described in the medical literature numerous times giving cause to a number of symptoms and diseases:
General headache
High blood pressure
Diabetes
Hypoxic pulmonary vasoconstriction
Cardiomyopathy
Pulmonary hypertonia with cor pulmonale (increased
pressure in the heart-lung circuits)
Heart failure, heart arrhythmia, heart attack
Day time melancholy or depression
Intelligence alterations
Acid Reflux (GERD - Gastro Esophageal Reflux Disease)
Potency disturbances
Worsening of ADHD (Attention Deficit Hyperactivity Disorder), in addition to a large number of problems of a more social character, like, e.g., divorce, decreased labour activity, difficulties in keeping conversations in the track due to tiredness, etc.

Thus, compared to a normal control group without diseases, patients suffering from snoring and/or OSAS appear to have: three times as many cases of coronary heart diseases, four times as many cerebral illnesses, such as clots, twelve times as many incidents of car accidents and twice as many labour accidents due to day time sleepiness as a result of lack of sleep and/or impaired sleep quality.

Due to these conditions the life time expectancy is severely limited for these patients, and their quality of life is compromised.

The continuum of snoring diseases gives the following frequency figures:
► 40% of adults over 40 snore (approx. 87 million Americans)
► 9% of men and 4% of women suffer from some form of OSAS (approx. 30 million Americans)
► Less than 10% of OSA sufferers have been diagnosed (Approx 3 million Americans)
► Of those, less than 25% have been successfully treated.

For the above reasons, it is important to provide devices to eliminate and prevent apnea and the incipient stages thereof.

In the prior art, a number of surgical techniques for removal of the tissue involved in the obstruction have been developed, but all of these techniques seem to incur a certain invalidation of the patient and, at the same time, do not have a fully predictable effect.

Furthermore, a number of medical treatments have been tried out with predominantly deficient or sometimes even damaging effect.

Finally, the scientific literature and the patent literature disclose numerous devices for alarming the snoring patient during sleep; devices for tongue thrust, devices for forward movement of the soft palate; devices for obstructing the oral cavity (delimited by the lips), thereby engaging the sound from the snoring; furthermore, mandibular advancement splints or appliances, mouth guard-like devices for provocation of either tongue, hyoid bone or jaw position changes, thereby eliminating snoring; - all of these requiring active participation from competent professionals, such as medical doctors, dentists, etc. Among such prior art devices for or attempts to inhibit snoring, the following are of particular interest in the present context:
EP 0 794 749 B1 (Ingemarsson-Matzen & Voss) discloses a jaw position-regulating oral device for preventing snoring and obstructive sleep apnea during sleep. The device consist of two members, a first member to engage with the maxillary dentition and a second member to engage with the mandibular dentition, both connected by a resilient hinge. The mechanism is embedded in the mandibular advancement relative to the maxilla. The main difference from the present patent application is the lack of adjustability of the length of the members relative to each other.

WO 2013 / 032 884 A1 (Fallon & Jung) discloses a mandibular advancement device with an upper and lower member to engage the maxillary and mandibulary dentition respectively. The lower tray assembly is mated to and slidable adjustable by the patient relative to the upper tray assembly. The main difference from the present patent application is that the device lacks a resilient hinge.

WO 2009 / 062 541 A1 (Magning & Magnin) discloses a mandibular advancement orthosis in which the device the comprises a unitary flexible member that can be folded on itself for interaction with the teeth of the upper and lower arches, and an interchangeable flexible strip for surrounding the teeth of the upper arch, having a length that can be modified in order to obtain the desired level of mandibular advancement. The main difference from the present patent application is that the device is regulated by detachable flexible strips and not intrinsic in the device itself.

US 2009 / 0014 013 A1 (Magnin) discloses a mandibular advancement splint made of two thermoformable trays designed to envelop the upper and lower arch. The advancement splint includes an articulated frame having rigid and flexible elements immersed in the thermoformable flexible material or molded around it. The main difference from the present patent application is that the device is made by two separate members without any hinge.

EP 1 719 481 A1 (Arni) discloses a mandibular advancement device with a lateral link incorporated into a mandibular protrusion device comprising an upper dental tray and a lower dental tray so as to advance or retract the lower dental arch during a vertical movement between the two. The link is adapted to be detachably accommodated in an opening of a ball pivot. The main difference from the present patent application is that the device lacks the intrinsic resilient hinge.

EP 2 529 710 A1 (Ash) discloses a device for mandibular advancement in which an upper member and a lower member are interconnected by means of pivotal connection in which at least one is formed as a stud. The main difference from the present patent application is that the device is made of two separate members with detachable attachment cylinders and clamps attached to the outside of the members.

CA 223 650 3 A1 (Frantz & Frantz) discloses a mandibular advancement device which uses elastic bands to pull the jaw forward. The upper part having a set of retention hooks and the lower part having a set of interchangeable slide-in posterior occlusal bite planes. The main difference from the present patent application is that the device is using of detachable elastic bands and no intrinsic resilience in any hinge.

WO 2008 / 130 413 A1 (Meade) discloses a mandibular advancement device for pulling the lower jaw forward composed of an upper and a lower member to engage the dentition, where a ball type of hook support is located on both sides of the upper tray at a forward position and a ball type of hook supports are located at a rearward position of both sides of the lower jaw. A tension coil is attached to each of the upper and lower ball type of hook supports. The main difference from the present patent application is that the device uses detachable hooks and spring coils and lack the resilient hinge mechanism.

US 2013 / 001 4765 A1 (Meade) discloses a mandibular advancement device for pulling the lower jaw forward composed of an upper and a lower member to engage the dentition, where a ball type of hook support is located on both sides of the upper tray at a forward position and a ball type of hook supports are located at a rearward position of both sides of the lower jaw. A tension coil is attached to each of the upper and lower ball type of hook supports. The main difference from the present patent application is that the device uses detachable hooks and spring coils and lack the resilient hinge mechanism.

WO 2011 / 115 962 A1 (Van Dyke & Tucker) discloses a mandibular advancement splint made of two trays designed to envelop the upper and lower arch. The upper appliance has a pair of adjustable wings attached to the body, and the lower has a pair of fixed wings attached to the body. The upper wings are slidable adjustable. The main difference from the present patent application is that the device is made of two separate members without any hinge and that it uses detachable pivots in both upper and lower members.

US 2010 / 004 380 5 A1 (Kelly) discloses a mandibular advancement device with an upper and lower member to engage with the dentition of the human. The lower dental plate having two pairs of spaced apart pillars and two removable attachable horizontal displacements inserts on the upper part. The main difference from the present patent application is that the device uses detachable vertical displacements inserts and lacks the resilient hinge mechanism.

GB 2 264 868 A (Mateljan) discloses an anti-snoring device for oral use, comprising members having upper and lower surfaces which engage the user's maxillary and mandibular dental arches respectively. The upper and lower surfaces are spaced so that the mandible is placed in a forwardly offset position relative to its normal position. The spacing also tensions the masticatory muscles to maintain the device in place. The main difference from the present patent application is that the device has no capability of adjustability in antero-posterior directions, and no hinge in the back part of the device.

US 2011 / 001 722 0 A1 (Lindsay et al.) discloses a self- titratable mandibular repositioning device that allows for adjusting the maintained forward position by simply biting-down to preserve the desired degree of mandibular advancement, made of a lower and an upper member to engage the dentition. The main difference from the present patent application is that the device has no resiliency or any hinge.

US 2008 / 011 579 1 A1 (Heine) discloses a mandibular advancement device with an intraocclusal removable device in the form of a "U" that is placed covering all of the upper jaw teeth, wherein two steps, one in each extreme of the lower part of the element, which impede the mandible be closed completely on its normal occlusion, forcing it to produce a forward displacement of the lower jaw. The main difference from the present patent application is that the device is a one member device with no hinge and only minor protrusive force can be applied to the lower jaw and only in occlusion.

US 2005 / 023 600 3 A1 (Meader) discloses a mandibular advancement device as a single piece of molded plastic with said unit modeled from four theoretical positions including a shield like anterior portion fitted and anchored between anterior teeth-gums and behind the lips. The main difference from the present patent application is that the device is a mono-block decided for prevention of lip closure.

US 2010 / 030 045 8 A1 (Stubbs et al.) discloses a mandibular advancement device with an upper and lower member to engage with the dentition of the human. The members are including a cam associated with one of the jaws and a follower associated with the other jaw. The main difference from the present patent application is that the device is lacking the hinge in the posterior part.

US 2008 / 009 902 9 A1 (Lamberg) discloses a mandibular advancement device composed of a maxillary main body for removable attachment to the maxillary teeth with a protrusive element extending from the central portion of the body and a mandibular removable appliance attached to the mandibular anterior teeth. The main difference from the present patent application is that the device is having a forcing mechanism on the lower jaw and lacks the posterior hinge.

EP 2 491 901 A1 (Garcia Urbano) discloses regulatable intraoral mandibular advancement device for preventing snoring and sleep apnea in which a screw system is located in the central part of the connection between the upper and lower members for the engagement of the dentition. The main difference from the present patent application is that the device is operated by a non intrinsic screw and screwdriver to maintain the regulated forward position of the mandible relative to the maxilla.

AU 1999 476 15 B2 (Palmisano) discloses a mandibular advancement device in which the upper jaw is firmly fitted into an upper plate and the lower jaw is firmly fitted into a lower plate, these two parts are connected by means of opposing flange components located to be lying in an area and close to the posterior teeth. The main difference from the present patent application is that the device is constructed with a pivotal mechanism and not a hinge.

US 2013 / 001 476 5 A1 discloses a tongue and mandibular advancement device in which an upper member has hook supports anteriorly and a lower member has a plurality of hook support at the rearward position. The main difference from the present patent application is that the device is constructed by two members separated system without any intrinsic hinge.

EP 0 337 201 discloses an orthodontic appliance comprising a first member to engage with the mandibular dentition and a second member to engage with the maxillary dentition. The two members are resiliently hinged together to keep the upper and lower jaw in a normal position. The main difference from the present patent application is that the device is lacking the adjustability in the sagittal plane, thereby delimitating the usability as a snoring preventing device.

WO 92/11827 discloses an anti-snoring device for oral use consisting of a horseshoe-like upper jaw member for engaging the maxillary dentition, with the downward extending flange intended to extend into the lingual vestibule in order to maintain a forward posture of the lower jaw. The main difference from the present patent application is that the device is using a forcing mechanism on the lower jaw. EP O 312 368 discloses an anti-snoring device for oral use which resembles the above-mentioned device, the main difference being the design of the airway passage. The main difference from the present patent application is that the device is using a forcing mechanism on the lower jaw.

WO 92/05752 (Wu) discloses an anti-snoring device for oral use consisting of a spatial member congruent with the palate and a lower member adapted to the lingual aspects of the surfaces of the dentition in the lower jaw, hooks being attached to the occlusive plane of the device for fixing the two jaws in a predetermined relation. The main difference from the present patent application is that the device is using detachable elastics and has no resilient hinge.

US 5,313,960 (Tomasi) discloses an anti-snoring device for oral use consisting of two horseshoe-like individually shaped mouthpiece portions which are connected and fixed in a predetermined position in which the lower jaw protrudes in relation to the upper jaw. The main difference from the present patent application is that the device is using a predetermined forward position of the lower jaw relative to the upper jaw, and there is no resilient hinge.

US 7,910,502 B1 (Nguyen & Nguyen) discloses an anti-snoring device for oral use consisting of two horseshoe-like individually shaped mouthpiece portions which are connected and fixed by an assembly of tubes, hooks and screws to be attached to the two separate members. The main difference from the present patent application is that the device is using detachable screws, spring coils and loops to keep the mandible in a forward position, meaning that there is no intrinsic resilient hinge. Therefore the device is much more complicated and technical demanding, with an additional disadvantage of plaque accumulation and deteriorated hygiene to follow.

DE 201 02 432 U1 (Trentepohl et al.) discloses an anti-snoring device for the oral use comprising an upper and lower member to engage with the maxillary and mandibulary dentition interconnected by an adjustable telescopic device which is attached at the outer surface of the members. The main difference from the present patent application is that the device is using detachable telescopic devices to keep the mandible in a forward position. Thus the device lacks the intrinsic resilient hinge. Also the device is much more complicated and technical demanding, with an additional disadvantage of plaque accumulation and deteriorated hygiene to follow.

WO 21013 049 751 A2 (Rogers), upon which the preamble of claim 1 is based, discloses a method for use in connection with sleep-disordered breathing, of forming oral orthotic systems to position and /or stabilize a mandible of a patient includes providing an upper dental member adapted to be placed in connection with upper dentition of the patient, providing a lower dental member adapted to be placed in connection with lower dentition of the patient providing a plurality of posterior mounting structures. Each of the posterior mounting structures is adapted to be attached to one of the upper dental member or the lower dental member at a posterior, buccal position thereon. Each of the posterior mounting structures includes a plurality of positions at which one of the pluralities of connectors is attachable to the extending member. Force may be applied to the mandible of the patient via at least one of a plurality of different mechanisms via attachment of a component of the mechanism to at least one of the posterior mounting structures. The upper dental member and the lower dental member are formed, independently, from at least one polymeric material. The main difference from the present patent application is that the device is using multiple detachable mounting structures to keep the mandible in a forward position. Thus the device lacks the intrinsic resilient hinge. The upper and lower members are constructed after direct impressions of the user's teeth, and then separately in a second laboratory procedure casts the hard polymeric (acrylic) material to form congruent trays for the upper and lower jaws. As this system is using hard acrylic material it substantially differ from the present patent application in function, durability and comfort. This gives a disadvantage in regard to monetary price for the end user. Also the device is much more complicated and technical demanding, with an additional disadvantage of plaque accumulation and deteriorated hygiene to follow.

US 2013 009 837 2A1 (Webster et al.) discloses an oral appliance for prevention of sleeping problems, including snoring, sleep apnea and bruxism. Specifically the device alters the position of the mandible and is made of a one piece device molded from a flexible polymer. Both upper and lower dental trays include inner and outer walls which increase contact area with the teeth. The hinge mechanism of the device includes a positive positioning system comprised of upper and lower opposed interlocking ridges. The ridges serve to create offset between the position of the upper and lower tray relative to each other, therefore advancing the user's mandible. The main difference from the present patent application is that the device is a one-piece device folded on a fixed point in the back most posterior part of the device to keep the mandible in a forward position. The interlocking ridges keep the lower and upper trays in a fixed forwarded position, thereby merely prohibits the other vice rolling possibility of the device leading into misuse of the intention of the device. Also there is no kind of incremental adjustability. Hence there is no individualization possibility.

US 2011 022 626 1A1 (Hernandez) discloses a mouthpiece for reducing snoring. The mouthpiece includes an upper guard configured to fit over the upper teeth of the user, a lower guard configured to fit over the lower teeth of a user, a spacer assembly provided between the upper guard or the lower guard to provide an air passage at the middle section of the mouthpiece, a first adjustable assembly attached to the left side of the upper guard and the left side of the lower guard, and a second adjustable assembly attached to the right side of the upper guard and the right side of the lower guard. The first adjustable assembly and the second adjustable assembly are operable to move the lower guard relative to the upper guard. The main difference from the present patent application is that the device is using detachable blocks and screws devices to keep the mandible in a forward position. Although the device depicts a kind of hinge (flexible coplanar connectors), this hinge is merely decided for avoiding sharp edges at the posterior part of the two members and for ease and economy of manufacture , and can not function as an actual durable active hinge due to the fact that the screws in the blocks are rigid. In conclusion, the device is much more complicated and technical demanding, with an additional disadvantage of plaque accumulation and deteriorated hygiene to follow.

WO 01 302 60 A1 (Bergersen) discloses a patent application as an intra-oral appliance for repositioning the user's mandible anterior to the user's maxillary teeth, thus opening the user's oral, pharyngeal passageway preventing snoring and sleep apnea. The appliance is two U- shaped plates joined to form a hinge. The lower plate has lingual tabs which are employed to help position the appliance. The main difference from the present patent application is that the device is completely lacking any kind of incrementally adjustability. Also the WO 01 302 60 A1 is merely a transcription of the original patent EP 0 794 749 B1 (Ingemarsson-Matzen & Voss) except the lingual tabs which in the original version is a solid moldable block.

### DISCLOSURE OF THE INVENTION

While the above devices represent attempts to solve the snoring and apnea problems, they are all rather complicated in their design and most of these require the interaction of a professional team in their individual design. Furthermore, they are rather discomfortable for the wearer, and they do not appear convincing with respect to their capability of achieving an effective and long-lasting anti-snoring effect.

Thus, there is a demand for a relatively comfortable device which provides a high degree of inhibitory effect on snoring during even long sleeping periods, such as overnight, without adverse effects on the structures involved, and which at the same time is easy and simple to use and wear for normal non-skilled persons.

The present invention provides such a device.

The adjustable anti-snore device according to the invention comprises an upper member adapted to engage the maxillary dentition of a human and a lower member adapted to engage the mandibulary dentition of the human, the upper and lower members being resiliently or mechanically hinged together, wherein the resiliency of the hinging is adapted to allow the physiological movement of the lower jaw in the sagittal plane while retaining a forward position of the lower jaw relative to the upper jaw and thereby keeping the airway passage in the nasopharynx, the oropharynx and the hypopharynx substantially free of occlusion, while at the same time embody the adjustability in one or two form, i.e. incremental and / or successive.

The device according to the invention combines three essential functions: the forward positioning of the lower jaw relative to the upper jaw, the hinging, and the adjustability of the sagittal relation between the two members. As will be explained below, the forward positioning of the lower jaw is essential to prevent occlusion of the airway passage in the pharyngeal space during sleep. The resilient or mechanically hinging makes it possible and realistic to maintain the forward positioning of the lower jaw even during movements in the sagittal plane which unavoidably occur during sleep. And the adjustability makes it useful for even the smallest and the largest person wearing the device. This essential combination of features which ensures constant non-constricted airflow and unrestricted movement in the sagittal plane and thereby ensures a constant efficient function without risk of the device falling out of the mouth of the user and without any substantial discomfort together with the adjustability which even allows some horizontal movements, distinguishes the device according to the invention from all of the abovementioned prior art devices.

The device according to the invention may be made of any material, such as metal, alloy, wood, plastics, etc. provided that the device made feels soft and comfortable in the mouth without any constriction or damaging of the tissue, such as gums, tongue, teeth, but at the same time is sufficiently capable of retaining its shape and of exerting a sufficient resiliency towards the muscular tension and forces acted upon the jaws so that it will maintain the lower jaw in the anterior position while allowing normal movements during sleep. The material used for the device according to the invention should not contain any allergens or other kind of toxic ingredients.

The device according to the invention is preferably made of a resilient non-toxic plastics material, such as a polyvinyl resin, including a vinyl acetate-ethylene copolymer such as poly (ethyl vinyl acetate), or a polyolefin such as polyethylene or polypropylene.

It is particularly preferred that the resilient non-toxic plastics material is a thermoplastic material, such as a cellulose derivative, a vinyl polymer, a polystyrene, a polyamide, an acrylic resin, etc., which can be shaped to adapt to an individual dentition by moderate heating, such as heating to a temperature above normal human body temperature, that is, a temperature of at least 40°C and at the most 80°C, e.g. about 70°C. The material presently most preferred by the inventor is ethylene vinyl acetate copolymer.

The device according to the present invention may be manufactured by plastics molding, such as cold molding, compression molding, injection molding, etc. The manufacturing method presently most preferred by the inventor is injection molding.

The upper and lower members are preferably integrated with each other through resilient hinges made of the same material as the upper and lower members. However, the hinges may be reinforced and their resiliency enhanced by insertion, such as cast in, etc., into the hinges of a resilient member, such as a resilient plastics member, a metallic resilient member, such as a flat spring, a laminated spring etc. etc., or simply by a mechanical connection.

The adjustability according to this invention, being embedded or glued, gilded or otherwise attached to the membering parts for the engagement of the dentition, in the upper maxillary member, the lower mandibulary member or both members at the same time, being incremental is a major novelty and is unique for this device.

As it will be understood the adjustability is embedded in the anterior parts of the upper and / or lower members of the device thus keeping the posterior resilient or mechanic hinge intact.

The adjustability of the protrusion of the mandible relative to the maxilla is made adjustable by the means of embedding a positive structure in either the forward moving part or the device and a negative structure in the stable part of the device or vice versa. The positive part may be constructed as a knob, rod, hook or alike, whereas the corresponding negative structures would be holes, cylinders and loops in this aspect. Other configurations may apply. Thus regardless on the structure selected, knob, rod or hook etc. the adjustability will express itself as an incremental (stepwise) adjustable mandibular advancement device.

One aspect of the invention is a device in which the incrementally adjustable mechanism is embedded in the mandibular part of the device so the mandible can be protruded when the knobs and holes are detached from its original position and reattached in a more advanced longitudinal form of the mandible part of the device.

Another aspect of the device is a device in which the incrementally adjustable mechanism is embedded in the maxillary incisal, canine or premolar region of the device so the maxilla can be retracted when the knobs and holes (or any of the other described adjustability forms) are detached from its original position and reattached in a more tightened position. In this way the circumference of the dental arch of the device will decrease. As the maxilla is not able to move, the result of this maneuver will be the forward displacement of the mandible from its original position.

A particularly preferred way of shipping the device according to the invention to the end consumer is as a kit comprising the device and a temperature indicator adapted to indicate a temperature change to an elevated temperature at which the material of the device can be shaped. This makes it simple and safe for the end user to mold the device to conform to his or hers specific dentition simply by heating the relevant domain of the device in water at the temperature of which is kept in the correct temperature range for the material in question by using the indication of the temperature indicator.

It should be understood that the use of the anti-snore device according to the invention is not limited to prevention or reduction of snoring or OSAS but the device is applicable in any situation where it is desirable to secure free airway passage in human beings, such as during recovery from anesthesia, during unconsciousness, etc.

The unique combination of posterior resilient / mechanic hinging, dentition engagement and adjustability discussed above can also, according to another aspect of the invention, be utilized in a device for relieving guided transpositions of the jaws. In this latter aspect, the invention relates to an orthognatic function device comprising an upper member adapted to engage with the maxillary dentition of a human and a lower member adapted to engage with the mandibulary dentition of the human, the upper and lower members having bases which prevent direct contact between opposing teeth, thereby eliminating guided transposition of the jaw relation and the upper and lower members being resiliently hinged together in such a manner together with the adjustability that the lower jaw of the human is kept positioned in a normal position relative to the upper jaw, allowing vertical movement and, in the occluded intercuspidal position, allowing horizontal movement so that the temporo mandibular joint is kept substantially in its resting position, both when the lower jaw is at rest and when it is working.

It will be understood that also in this aspect, the resiliency of the hinging should be adapted to allow the physiological movement of the lower jaw in the sagittal plane, and that the above comments concerning selection of suitable materials, manufacturing method, and adaptation to the individual dentition by shaping the material in a softened, e.g. heat-softened, condition apply also to this aspect of the invention.

Thus, the orthognatic aspect of the invention provides a completely new philosophy in relieving temporo mandibular joint disorders caused by irregular dentition: In contrast to known orthognatic devices, the orthognatic device according to the invention is hinged in such a way that no interference between the upper and lower members can occur, thereby alleviating symptoms caused by abnormal interference from irregular dentition. Furthermore, tensions caused by tooth grinding and clenching are alleviated. At the same time, this orthognatic device is much simpler to adapt to the individual needs of the person in question than conventional orthognatic devices, and it can even be used by the individual consumer without assistance by any professional.

In the following, the incremental mandibular advancement anti-snoring device aspect of the invention will be explained in further detail with reference to the accompanying drawings.

### DESCRIPTION OF THE USE OF THE DEVICE ACCORDING TO THE INVENTION

The preferred method of using the device described above and in the figures is to insert the device in the mouth of the affected individual, at nighttime before sleep.

By inserting the device in the mouth in the way that the mandible is forced a bit forward relative to the maxilla, increased airway space will appear in the back of throat, the pharynx, and thereby facilitate the free flow of air with its oxygen for the bodily metabolism at large.

Sometimes the alignment of the teeth is not congruent with the ideal shape of a perfect dentition, and therefore the need for special modifications can prevail. To accomplish this task, the preferred material used for the device according to this invention, is made of a thermoplastic material, which can be subjectively moulded to adapt more perfect to the users non-perfect alignment of the teeth. Simply by immersing that actual part of the device, that needs modulation, into water at a prefixed temperature according to the material specification, the material can be moulded and hence get in closer contact with alignment of the wearers dentition. When it returns to the temperature of the room, or inside the mouth, the device will keep its new dimension, and thereby alleviate any hard or any loose contact with the teeth, thereby making it much more comfortable to wear during the sleep.

This procedure can be done by almost everybody with a little exercise, and does not need to acquire the competence and time from a professional dentist, doctor or technician at all.

One aspect of the incremental mechanism of the device according to the invention is that the wearer of the device can be given numerous possibility to calibrate his own degree of forwarding the mandible relative to the maxilla, by the use of any or all of the incrementally mechanisms described above.

For instance if the wearer of the device wants to elongate the mandible, simply detach the device parts, find a new location for the parts relative to each other and then attach again. In one particular case the mandible part can be detached from the maxillary part integrated with the hinge, forwarded or retracted from the previous position, then reattach the snap-on mechanism, and the effect of the device will change, with the result of more or less free airway space in the pharynx (depending on weather the user elongated or diminished the relative length).

In another particular case the maxillary part can be detached from the mandibulary part integrated with the hinge, forwarded or retracted from the previous position, then reattach the snap-on mechanism, and then again the effect of the device will change.

Yet in another particular case the maxillary part can be adjusted one or more steps in one side of the device (right or left), and the mandibular part of the opposing side (left or right) can be adjusted one or more steps individually from the other side. This part of the device modification can give the benefit of alignment to dentitions that are asymmetric or any other kind of special needs.

In some cases the user of the invention would prefer the incremental mechanism just to be situated in the front midline of the maxillary part of the device, and then for this is expressed through the description and the drawings. If the midline incremental mechanism is opened (detached snap-off) the circumference of the maxilla can be enlarged or diminished. If the arch circumference is enlarged relative to the mandible, the mandible is falling back towards its normal (airway occluding) position, whereas if the arch circumference is diminished the mandible is being forced further forward and thereby reliefs the occluded airway passage.

Any of the above alterations of the position between the two jaws, can be made with or without the use of the temperature sensitive alteration of the thermoplastic materials.

Another aspect of the device according to the invention is that the wearer of the device can fix the relative position of the maxillary part relative to the mandibulary art by the use of heating, either from the warmed water or from the metal rod that can be provided in one way of delivering the device package. When the wearer have accomplished the desired position he can immerse the device into the heated water and fix the position there by locking the negative and positive structures to each other, or he can use the metal rod to melt a part of the connected devise to prevent it from leaving the accomplished and desired position.

### DESCRIPTION OF FIGURES

In the figures and drawings in which,
- Fig. 1: Shows the Incremental version of the Adjustable Mandibular Advancement Device top view in an oblique perspective in its Neutral position
- Fig. 2: Shows the Incremental version of the Adjustable Mandibular Advancement Device bottom view in an oblique perspective in its Neutral position
- Fig. 3: Shows the Incremental version of the Adjustable Mandibular Advancement Device bottom view in a perpendicular perspective in its Neutral position
- Fig. 4: Shows the Incremental version of the Adjustable Mandibular Advancement Device top view in a perpendicular perspective in its Neutral position
- Fig. 5: Shows the Incremental version of the Adjustable Mandibular Advancement Device from aside in its Neutral position
- Fig. 6: Shows the Incremental version of the Adjustable Mandibular Advancement Device in cross section at line A in fig. 3 in its Neutral position
- Fig. 7: Shows the Incremental version of the Adjustable Mandibular Advancement Device in cross section at line B in fig. 3 in its Neutral position
- Fig. 8: Shows enlarged schematic details of the incremental mechanism with taps and corresponding holes
- Fig. 9: Shows the Incremental version of the Adjustable Mandibular Advancement Device top view in an oblique perspective in its maximal elongated position
- Fig. 10: Shows the Incremental version of the Adjustable Mandibular Advancement Device bottom view in an oblique perspective in its maximal elongated position
- Fig. 11: Shows the Incremental version of the Adjustable Mandibular Advancement Device bottom view in a perpendicular perspective in its maximal elongated position
- Fig. 12: Shows the Incremental version of the Adjustable Mandibular Advancement Device top view in a perpendicular perspective in its maximal elongated position
- Fig. 13: Shows the Incremental version of the Adjustable Mandibular Advancement Device from aside in its maximal elongated position
- Fig. 14: Shows the Incremental version of the Adjustable Mandibular Advancement Device in cross section at line A in fig. 11 in its maximal elongated position
- Fig. 15: Shows the Incremental version of the Adjustable Mandibular Advancement Device in cross section at line B in fig. 11 in its maximal elongated position
- Fig. 16: Shows the Incremental version of the Adjustable Mandibular Advancement Device in its four components top view oblique perspective

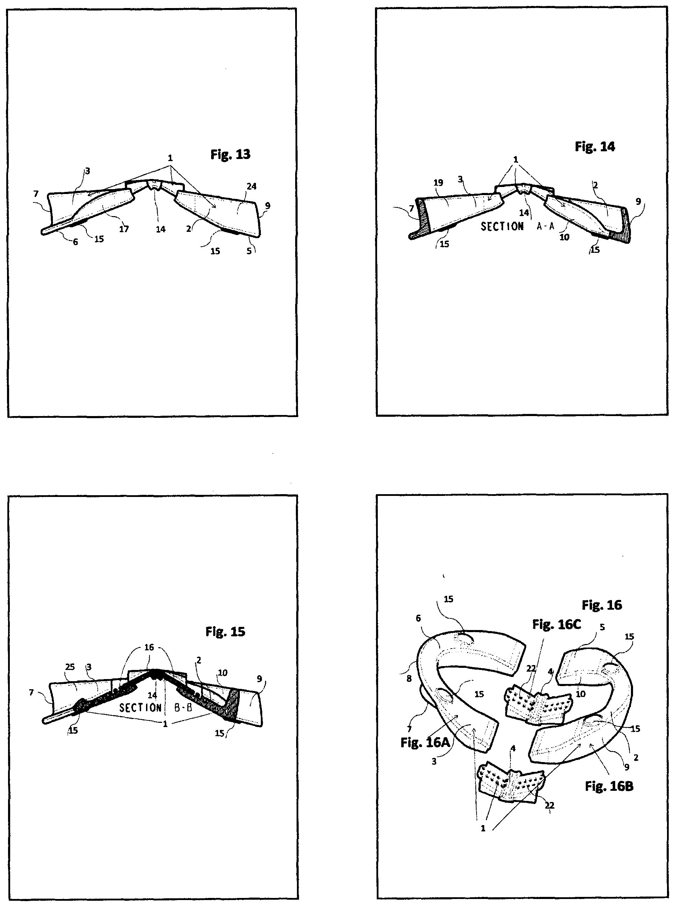

- Fig. 16 a: Shows the mandibulary lower member to engage with the maxillary dentition of the Incremental version of the Adjustable Mandibular Advancement Device top oblique view
- Fig. 16 b: Shows the maxillary upper member to engage with the mandibular dentition of the Incremental version of the Adjustable Mandibular Advancement Device top oblique view
- Fig. 16 c: Shows the Hinge mechanism connecting the upper maxillary and lower mandibulary members of the Incremental version of the Adjustable Mandibular Advancement Device in a top oblique view.

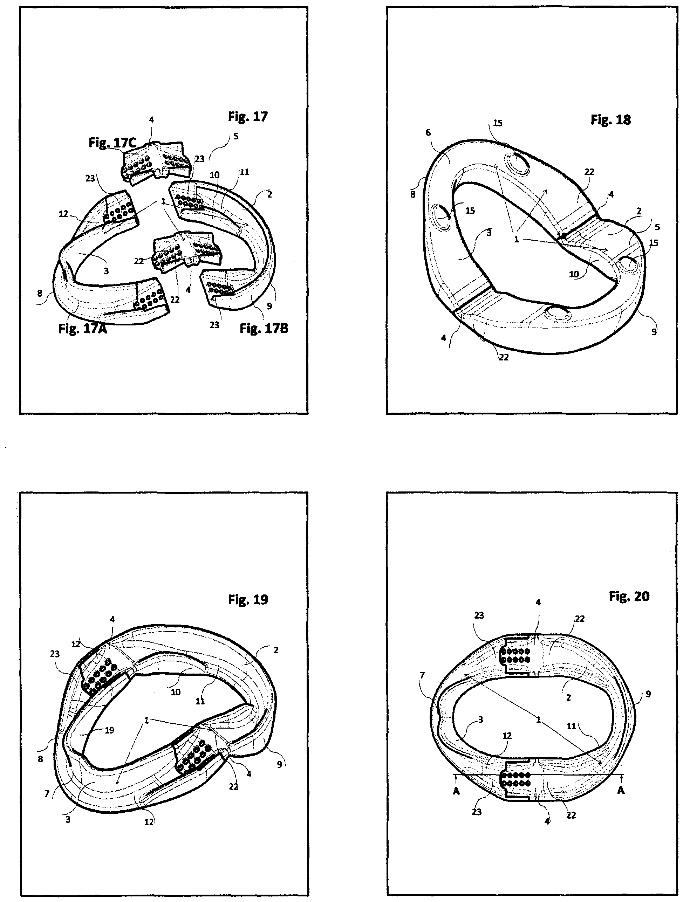

- Fig. 17: Shows the Incremental version of the Adjustable Mandibular Advancement Device in its four components bottom view oblique perspective
- Fig. 17 a: Shows the mandibular lower member to engage with the mandibular dentition of the Incremental version of the Adjustable Mandibular Advancement Device bottom view oblique perspective
- Fig. 17 b: Shows the maxillary upper member to engage with the maxillary dentition of the Incremental version of the Adjustable Mandibular Advancement Device bottom view oblique perspective
- Fig. 17 c: Shows the Hinge mechanism connecting the upper maxillary and lower mandibulary members of the Adjustable Mandibular Advancement Device in a bottom oblique view
- Fig. 18: Shows the single member adjustable Incremental version of the Adjustable Mandibular Advancement Device top view in an oblique perspective in its Neutral position
- Fig. 19: Shows the single member adjustable Incremental version of the Adjustable Mandibular Advancement Device bottom view in an oblique perspective in its Neutral position
- Fig. 20: Shows the single member adjustable Incremental version of the Adjustable Mandibular Advancement Device bottom view in a perpendicular perspective in its Neutral position

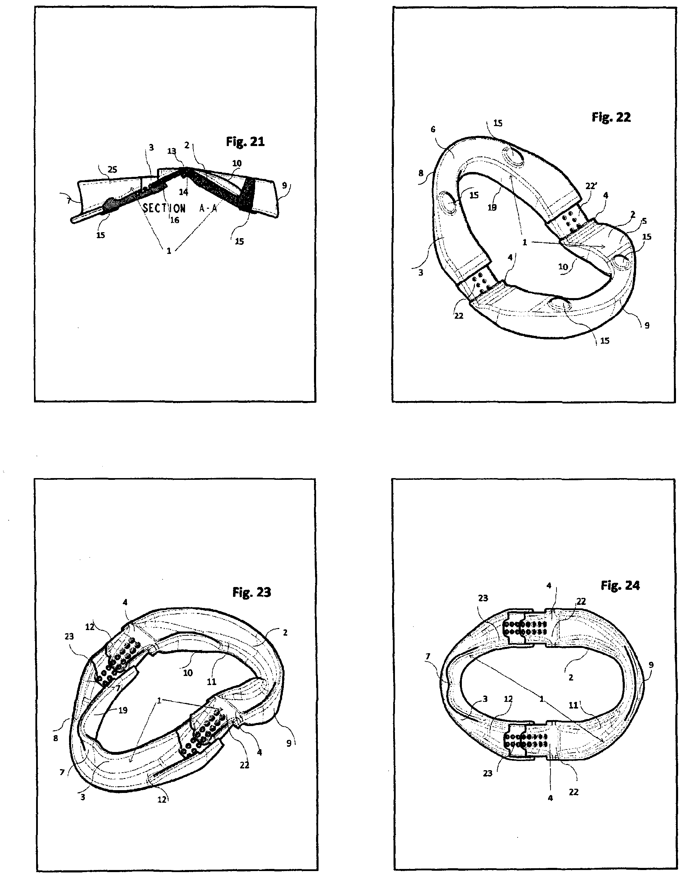

- Fig. 21: Shows enlarged schematic details of the incremental mechanism with taps and corresponding holes in just one of the members at line A-A in fig. 20 in its maximal elongated position
- Fig. 22: Shows the single member adjustable Incremental version of the Adjustable Mandibular Advancement Device top view in an oblique perspective in its maximal elongated position
- Fig. 23: Shows the single member adjustable Incremental version of the Adjustable Mandibular Advancement Device bottom view in an oblique perspective in its maximal elongated position
- Fig. 24: Shows the single member adjustable Incremental version of the Adjustable Mandibular Advancement Device bottom view in a perpendicular perspective in its maximal elongated position

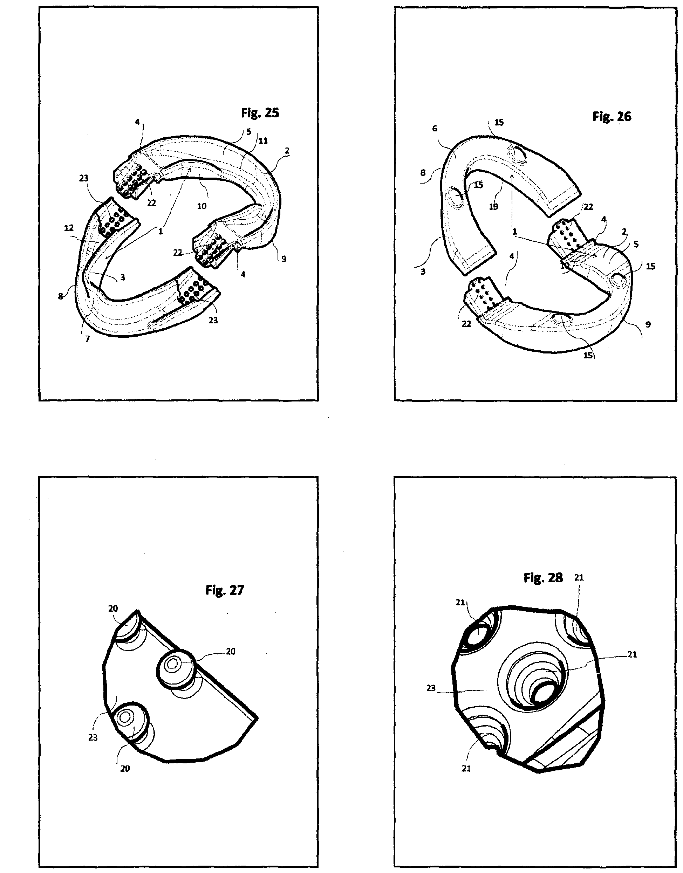

- Fig. 25: Shows the single member adjustable Incremental version of the Adjustable Mandibular Advancement Device bottom view in an oblique perspective with detached members
- Fig. 26: Shows the single member adjustable Incremental version of the Adjustable Mandibular Advancement Device top view in an oblique perspective with detached members
- Fig. 27: Shows in detail the knob part of the incremental mechanism
- Fig. 28: Shows in detail the hole part of the incremental mechanism

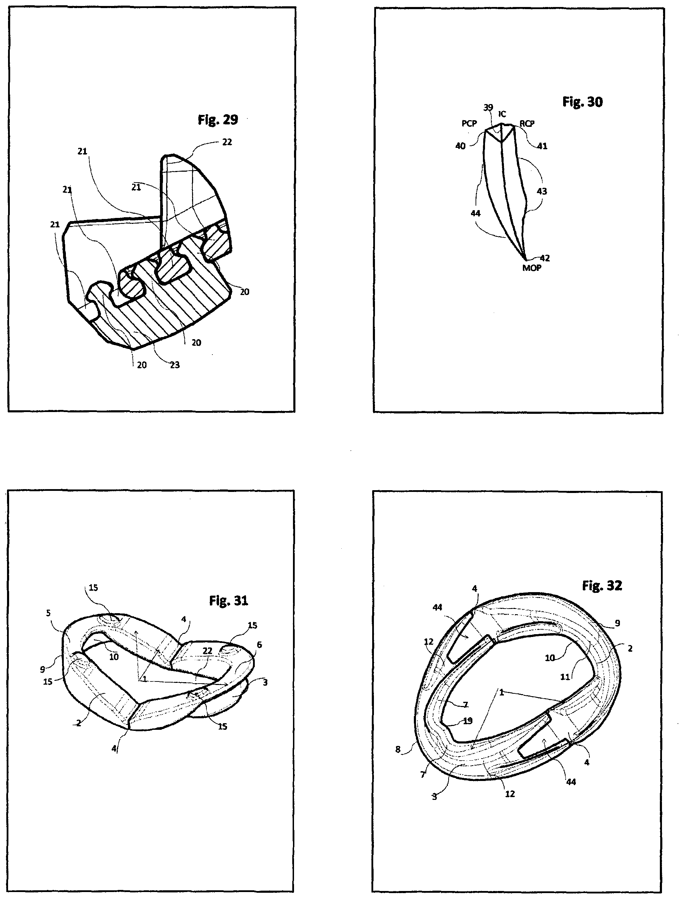

- Fig. 29: Shows a cross section of the griping mechanism in its maximum enlarged stage
- Fig. 30: Shows a diagrammatic representation of the limitations of the movements of the lower jaw in any direction in the sagittal plane where PCP stands for the most protruded contact point of the teeth, IC stands for intercuspidal position (the maximal closing point), RCP stands for the most retracted contact position for the teeth, and MOP stands for the maximal opening point.
- Fig. 31: Shows the single member Saw-tag Incremental version of the Adjustable Mandibular Advancement Device top view in an oblique perspective in its Neutral position. Note that the essential aspect of the Saw-tag incremental version of the Incrementally Adjustable Mandibular Advancement Device is the fact that the negative structures is lying IN the material and does not penetrate the material as in the previously described version of the Incrementally Adjustable Mandibular Advancement Device.
- Fig. 32: Shows the single member Saw-tag Incremental version of the Adjustable Mandibular Advancement Device bottom view in an oblique perspective in its Neutral position.

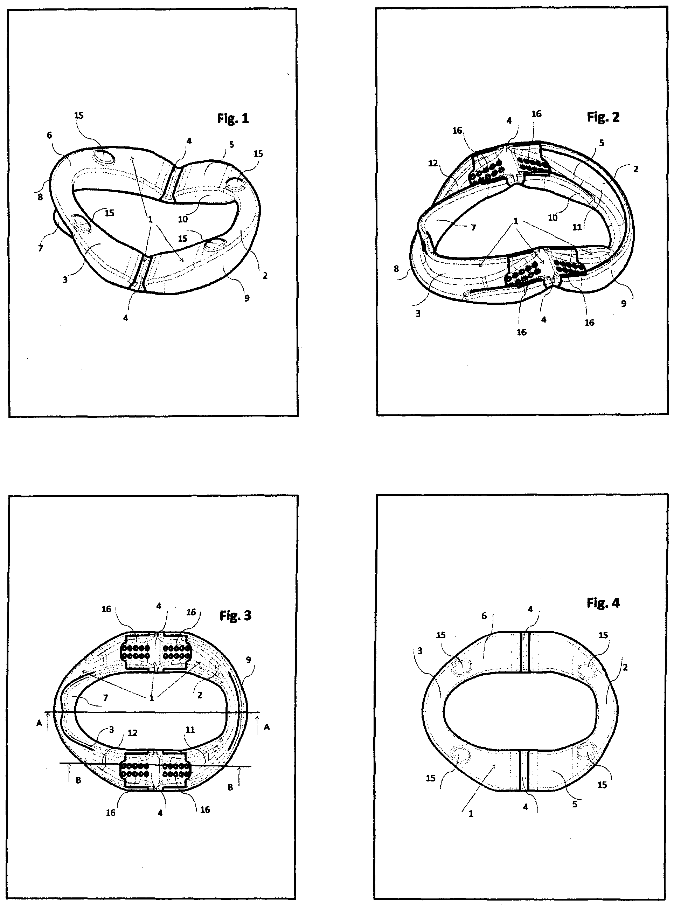

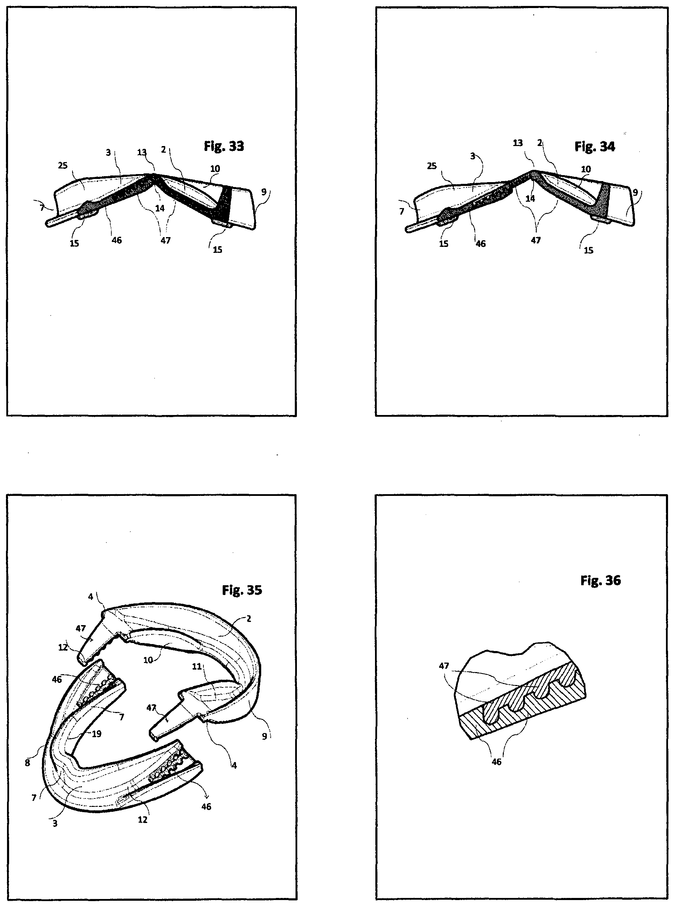

- Fig. 33: Shows the single member Saw-tag Incremental version of the Adjustable Mandibular Advancement Device in cross section at line B in fig. 3 in its Neutral position.
- Fig. 34: Shows the single member Incremental version of the Adjustable Mandibular Advancement Device in cross section at line B in fig. 3 in its maximal elongated position.
- Fig. 35: Shows the single member adjustable saw-tag Incremental version of the Adjustable Mandibular Advancement Device bottom view in an oblique perspective in its detached position.
- Fig. 36: Shows enlarged schematic details of the saw-tag incremental mechanism with positive embedded tabs in the maxillary part and corresponding embedded cavities in mandibular part of the invention.

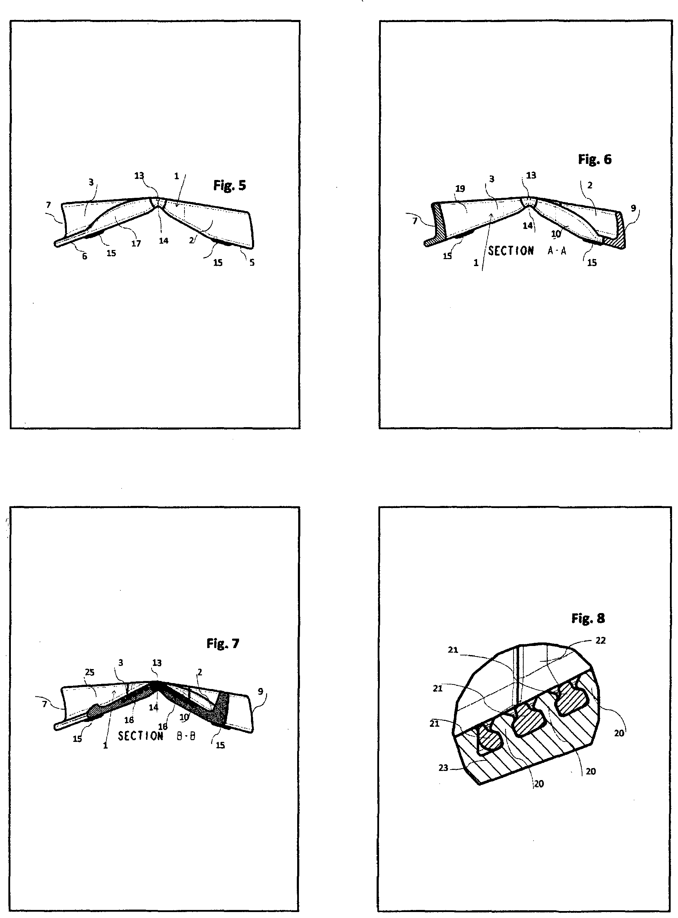

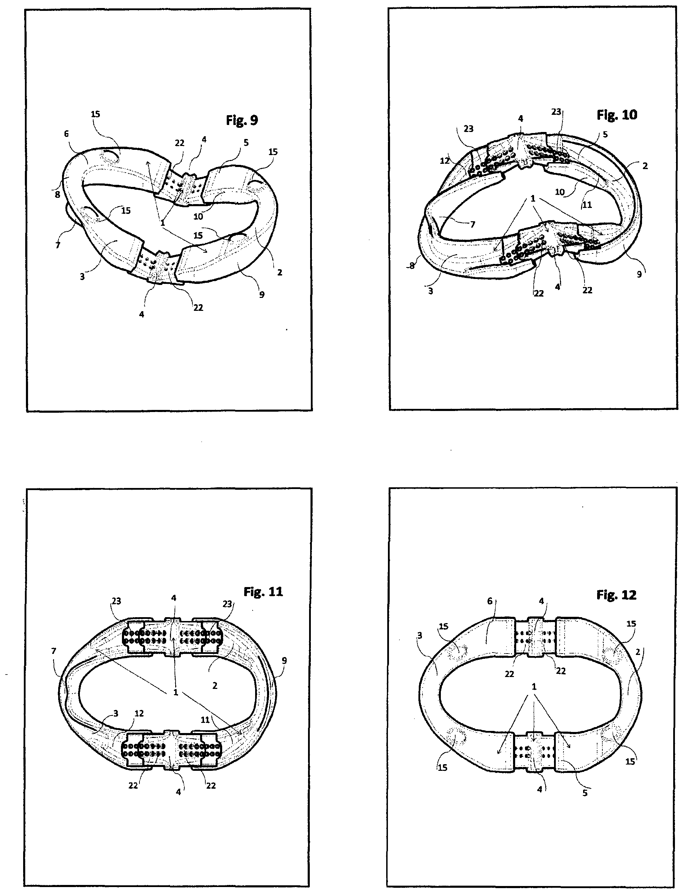

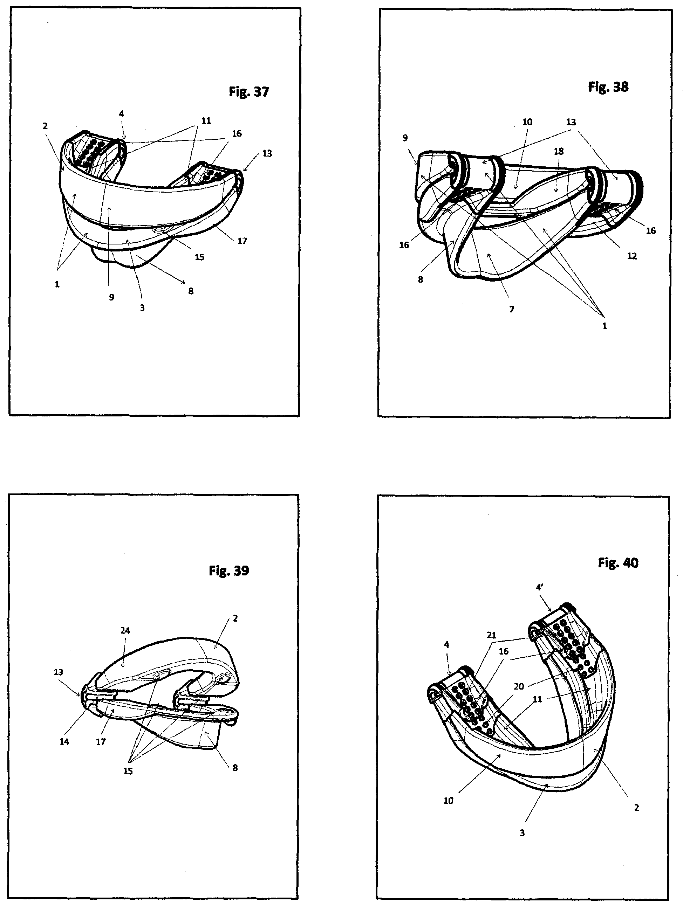

- Fig. 37: Shows the activated view of the three piece version of the Incremental version of the Adjustable Mandibular Advancement Device shown in figs. 1 through 6, and 9-17 in an oblique frontal upper view.
- Fig. 38: Shows the activated view of the three piece version of the Incremental version of the Adjustable Mandibular Advancement Device shown in figs. 1 through 6, and 9-17 in an oblique dorsal lower view.
- Fig. 39: Shows the activated elongated view of the three piece version of the Incremental version of the Adjustable Mandibular Advancement Device shown in figs. 1 through 6, and 9-17 in an oblique frontal view.
- Fig. 40: Shows the activated elongated view of the three piece version of the Incremental version of the Adjustable Mandibular Advancement Device shown in figs. 1 through 6, and 9-17 in an oblique almost cranial view.

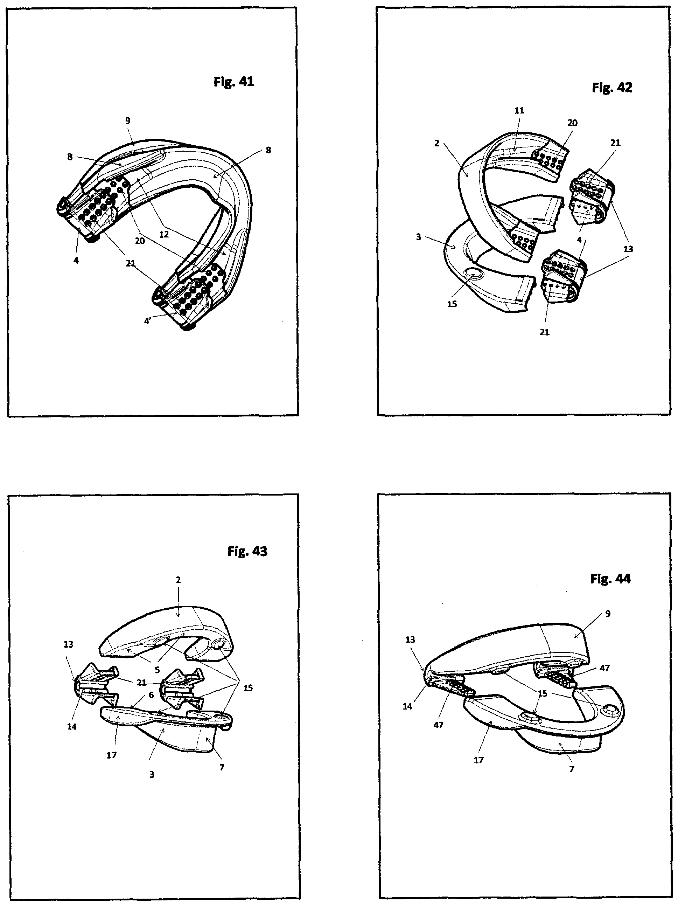

- Fig. 41: Shows the activated elongated view of the three piece version of the Incremental version of the Adjustable Mandibular Advancement Device shown in figs. 1 through 6, and 9-17 in an oblique bottom view.
- Fig. 42: Shows the activated elongated view of the three piece version of the Incremental version of the Adjustable Mandibular Advancement Device shown in figs. 1 through 6, and 9-17 in an oblique lateral view detached.
- Fig 43: Shows the activated elongated view of the three piece version of the Incremental version of the Adjustable Mandibular Advancement Device shown in figs. 1 through 6, and 9-17 in an oblique almost frontal view detached.
- Fig. 44: Shows the activated elongated view of the saw-tag version of the Incremental version of the Adjustable Mandibular Advancement Device shown in figs. 31 through 35, in an oblique almost frontal view detached.

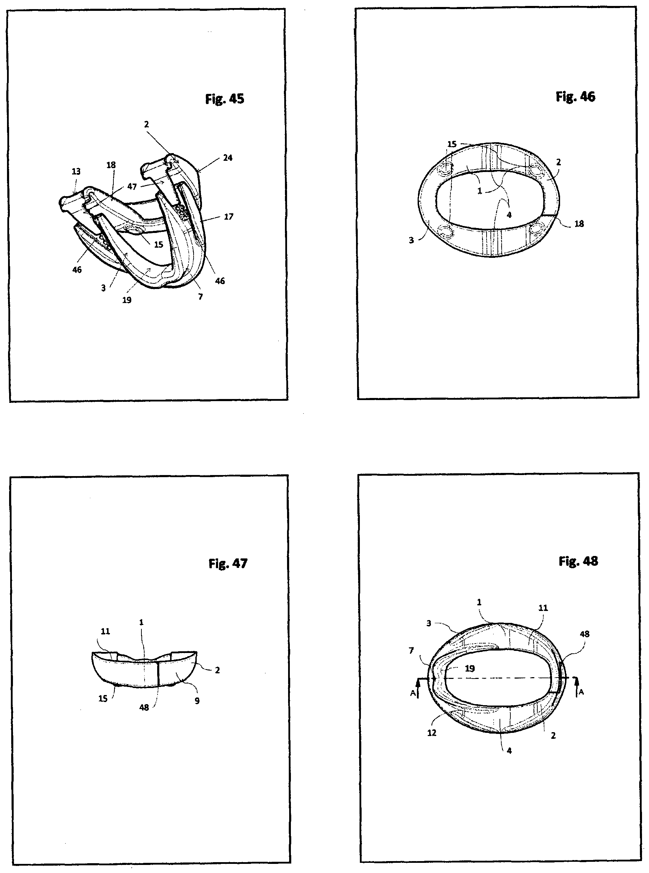

- Fig. 45: Shows the activated elongated view of the saw-tag version of the Incremental version of the Adjustable Mandibular Advancement Device shown in figs. 31 through 35, in a bottom dorsal oblique perspective detached.
- Fig. 46: Shows the Midline Maxillary Incremental version of the Adjustable Mandibular Advancement Device top view in a perpendicular perspective in its Neutral position.
- Fig. 47: Shows the Midline Maxillary Incremental version of the Adjustable Mandibular Advancement Device front view in its Neutral position (mandibular part not shown here).
- Fig. 48: Shows the Midline Maxillary Incremental version of the Adjustable Mandibular Advancement Device bottom view in a perpendicular perspective in its Neutral position.

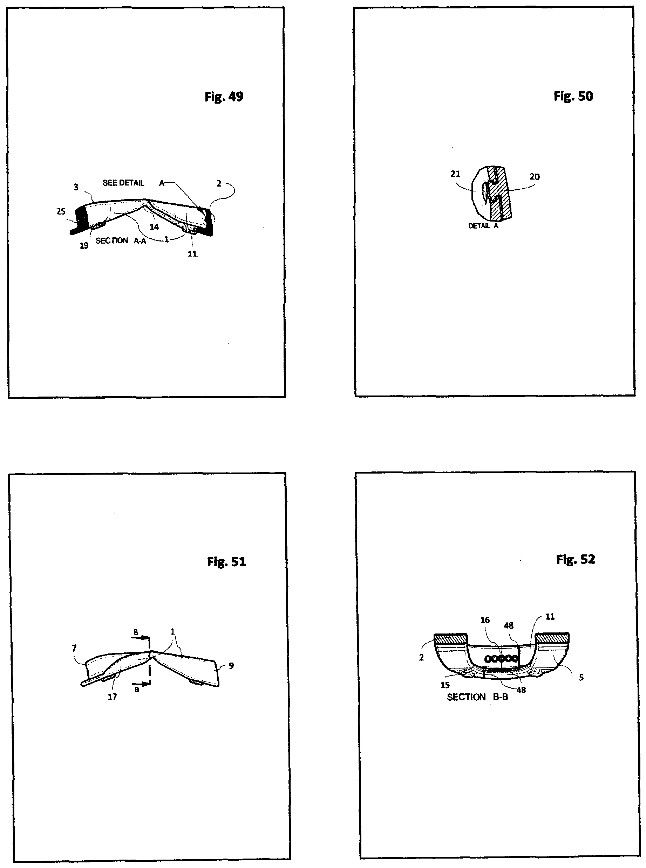

- Fig. 49: Shows the Midline Maxillary Incremental version of the Adjustable Mandibular Advancement Device sagittal trans-sectional view in the midline shown as line A-A in fig. 48, in its Neutral position.
- Fig. 50: Shows the incremental mechanism with its positive and negative structures interconnected. It can be seen that the two slices of the facial wall of the maxillary part, when joined, exhibits as the original facial wall as seen in figs. 1 - 7, 9 -26, 31 - 35, and 37 - 45.
- Fig. 51: Shows the Midline Maxillary Incremental version of the Adjustable Mandibular Advancement Device side view in a perpendicular perspective in its Neutral position.
- Fig. 52: Shows the Midline Maxillary Incremental version of the Adjustable Mandibular Advancement Device in a dorsal view in its Neutral position. The incremental mechanism is shown in its natural non-elongated position.

### DETAILED DESCRIPTION OF THE NOMENCLATURE USED IN THE DESCRIPTION OF THE FIGURES

In Figs. 1 through 45, in which like numerals indicate like parts, the device 1 according to the invention consists of two horseshoe-like members 2 and 3 of a soft, resilient plastics material, preferably a thermoplastic material, such as an ethylene vinyl acetate copolymer, or any suitable material, hinged together by means of integrated resilient or mechanical hinges 4 and 4'. Surfaces 5 and 6 represent the lower surface of the upper member (2) and the upper surface of the lower member (3), respectively. 7 indicates the lingual flange of the lower member (3) adapted to the lingual surfaces of the lower incisors, canines and premolars, this flange 7 being the part of the device which actually forces the lower jaw forward. 8 is the facial surface of the lower member (3), 9 is the facial surface of the upper member (2), and 10 is the lingual surface of the upper member (2). The conjoining effect of forces exerted by the facial surface 9 of the upper member (2) and the lingual surface 7 of the lower member (3) keep the lower jaw in a forward position relative to the upper jaw. As the facial surface 8 and the lingual surface 10 do not exert any forces, their dimensions are rather uncritical and some embodiments of the invention may even be provided without these surfaces. No. 11 indicates the tunnel shaped space of the upper member (2) to engage with the upper dentition and 12 indicates the tunnel-shaped space of the lower member (3) adapted to engage the lower dentition. When the device is compressed to an active position convexity 13 appears at the posterior end part of the device and a concavity 14 at the anterior part of the hinging. The occusal knots, 15 are designed to prevent complete occlusion and lack of airspace between the members 2 and 3. No. 16 indicates the incremental mechanism, 17 is the Lateral facial portion of the lower mandibular member (3) at the molar and premolar area, 18 is the Medial palatine portion of the upper maxillary member (2) at the molar and premolar area, 19 shows the Lingual wall of mandibular member (3) closest to the tongue. 20 is the Positive structure of the incremental mechanism, 21 the Negative structure of the incremental mechanism, 22 the Hinge member part of the invention with its negative structures of the incremental mechanism. 24 is the Lateral facial portion of the maxillary member (2) at the molar and premolar area, 25 the Lingual wall of mandibular member (3) closest to the teeth. No. 39 is the intercuspidal position (IP) in which the dentition of the mandible makes the maximal interference with the dentition of the maxilla; 40 is the protruded contact position (PCP) in which the mandible has made the maximal protruded movement from the IP position, still keeping some contact with the dentition of the maxilla; 41 is the retracted contact position (RCP) in which the mandible have made the maximal retraction from the IP position, still keeping some contact with the dentition of the maxilla, and 42 is the maximal opening point (MOP) in which the mandible has made the maximal opening movement from the IP position, all of which only being restricted by the muscles, the teeth, the ligaments and the discus involved in the temporo-mandibular joint system. 42 indicates the border describing the curve in which the mandible can slide open from the RCP, 43 is the border describing the curve in which the mandible can slide open from the PCP. No. 44 shows the Bottom view of the mandibular part of the saw-tag attachment structure and 45 is the Top view of the mandibular part of the saw-tag incremental mechanism, 46 is the Mandibular member part of the invention with its negative structure for engaging with the positive maxillary part structures of the saw-tag incremental mechanism and 47 is the Maxillary member part of the invention with its positive structure for engaging with the negative mandibulary parts structures of the saw-tag incremental mechanism. 48 is indicating the section line between the sliced part of the maxillary facial band in the closest proximity to the maxillary dentition especially in the region from the premolars in the right side to the premolars in the left side.

## Claims

1. An incremental mandibular advancement anti-snoring and obstructive sleep apnea preventing device (1) comprising an upper member (2) adapted to engage the maxillary dentition of a human and a lower member (3) adapted to engage the mandibulary dentition of a human, which members (2, 3) are incrementally adjustable to each other, **characterized in that** the upper (2) and lower (3) members are resiliently hinged together by posterior resilient hinges (4, 4') which are integrated with the members (2, 3) and located in the most posterior part of the device whereby the hinging is adapted to allow physiological movements of the lower jaw in the sagittal plane while retaining a forward position of the lower jaw relative to the upper jaw and thereby keeping the airway passage in the nasopharynx, the oropharynx and the hypopharynx substantially free of occlusion.

2. A device (1) according to claim 1 in which the lower member (3) is incrementally adjustable to the upper member (2) or in which the upper member (2) is incrementally adjustable to the lower member (3).

3. A device (1) according to claim 1 in which the incremental mechanism is embedded in bodies of the upper (2) and lower (3) members, in the body of the lower member (3), or in the body of the upper member (2).

4. A device (1) according to any of the claims 1-3 in which the incremental mechanism is made up of multiple stepwise "snap-on - snap-of" structures embedded in or attachable to the bodies either the upper (2) or lower (2) members or both at the same time.

5. A device (1) according to claim 4 in which the multiple stepwise incremental "snap-on - snap-of" structures is made by male versus female parts of different forms such as round, square, hexagonal and so on, designed to engage with its corresponding opposite part of female structural form.

6. A device (1) according to claim 5 in which the multiple stepwise incremental male snap-on structures are lobed with variable numbers of segments or in which the multiple stepwise incremental female snap-on structures are lobed with variable numbers of segments.

7. A device (1) according to claims 1 or 2 wherein the incremental mechanism is configured to be removed from the upper member (2), lower member (3) and hinges (4, 4'), renewed, restored and reattached to the upper member (2), lower member (3) and hinges (4, 4') again.

8. A device (1) according to claims 5-7 where the female structures of either the upper (2) or lower (3) member is adapted to be completely punched out of either the upper (2) or lower (3) member to correspond with the opposing member.

9. A device (1) according to claims 1 or 2 wherein the upper member (2) has an anterior wall adapted to be in contact with the facial surfaces of the incisors, canines and premolars of the upper jaw and lacks lingual palatal walls to adapt to the palatal surfaces of the upper teeth, and a lower member (3) having a posterior wall adapted to be in contact with the lingual surfaces of the incisors, canines and premolars of the lower jaw and lacking facial walls to engage with the facial surfaces of the lower teeth.

10. A device (1) according to any of the preceding claims wherein the upper member (2) and the lower member (3) is made of a non-toxic plastics material which is a thermoplastic material which can be shaped to adapt to an individual dentition by moderate heating.

11. A device (1) according to claims 1 or 2 wherein the upper and lower members (2, 3) are integrated with each other through resilient hinges (4, 4') made of the same material as the upper and lower members (2, 3).

12. A device (1) according to claims 1 or 2 wherein the hinges (4, 4') are located in the most dorsal part of the device (1) being embedded in or resilient due to the material itself.

13. A device (1) according to claims 11 or 12 in which the most dorsal part of the hinges (4, 4') is convex at the most posterior part and concave at the most anterior part of the hinges (4, 4') when situated in the patient's mouth.

14. A device (1) according to claims 1 or 2 wherein the incremental mechanism is embedded in the maxillary middle most frontal part of the device (1) closest to the teeth from left side premolar to right side premolar when situated in the patient's mouth.

15. A kit comprising a device (1) according to any of the preceding claims together with a temperature indicator adapted to indicate the temperature change to an elevated temperature at which the material of the device (1) can be shaped individually, and with a heatable metal rod combined with an insulating hand piece for the use of locking the sliding incremental process by means of penetration both members at the desired forward positioned upper member (2) relative to the lower member (3).

## Patentansprüche

1. Inkrementelle mandibulär Vorschubvorrichtung (1) zur Anti-Schnarchen und Verhinderung von obstruktiver Schlafapnoe, umfassend ein oberes Element (2), das zum Eingreifen in das Oberkiefergebiss eines Menschen angepasst ist, und ein unteres Element (3), das zum Eingreifen in das Unterkiefergebiss eines Menschen angepasst ist, welche Elemente (2,3) inkrementell zueinander verstellbar sind, **dadurch gekennzeichnet, dass** das obere (2) und das untere (3) Element durch hintere Scharniere (4, 4'), die in die Elemente (2,3) integriert sind, elastisch gelenkig miteinander verbunden sind , und befindet sich im hintersten Teil der Vorrichtung, wobei das Scharnier angepasst ist, um physiologische Bewegungen des Unterkiefers in der Sagittalebene zu ermöglichen, während eine Vorwärtsposition des Unterkiefers relativ zum Oberkiefer beibehalten wird und dadurch der Atemwegsdurchgang im Nasopharynx, der Oropharynx und der Hypopharynx im Wesentlichen okklusionsfrei beibehalten wird.

2. Vorrichtung (1) nach Anspruch 1, bei der das untere Element (3) inkrementell auf das obere Element (2) einstellbar ist oder bei der das obere Element (2) inkrementell auf das untere Element (3) einstellbar ist.

3. Vorrichtung (1) nach Anspruch 1, bei der der Inkrementalmechanismus in die Körper der oberen (2) und unteren (3) Elemente, in den Körper des unteren Elements (3) oder in den Körper des oberen Elements (2) eingebettet ist.

4. Vorrichtung (1) nach einem der Ansprüche 1 bis 3, bei der der Inkrementalmechanismus aus mehreren schrittweisen "Snap-on-Snap-of" -Strukturen besteht, welche sind eingebettet in die Körper, entweder der oberen (2) oder unteren (3) Elemente oder beide gleichzeitig, oder an diesen befestigt werden können.

5. Vorrichtung (1) nach Anspruch 4, bei der die mehrfachen schrittweisen inkrementellen "Snap-On-Snap-Of" -Strukturen durch männliche gegen weibliche Teile verschiedener Formen wie rund, quadratisch, sechseckig usw. hergestellt sind, die dazu bestimmt sind sich mit dem entsprechenden entgegengesetzten Teil der weiblichen Strukturform auseinandersetzen.

6. Vorrichtung (1) nach Anspruch 5, bei der die mehreren schrittweisen inkrementellen männlichen Snap-On-Strukturen mit variabler Anzahl von Segmenten gelappt sind oder bei der die mehreren schrittweisen inkrementellen weiblichen Snap-On-Strukturen mit variabler Anzahl von Segmenten gelappt sind.

7. Vorrichtung (1) nach Anspruch 1 oder 2, wobei der Inkrementalmechanismus so konfiguriert ist, dass er von dem oberen Element (2), dem unteren Element (3) und den Scharnieren (4, 4') entfernt, erneuert, wiederhergestellt und wieder an das obere Element (2), das untere Element (3) und die Scharniere (4, 4') gebracht werden kann.

8. Vorrichtung (1) nach den Ansprüchen 5 bis 7, wobei die weiblichen Strukturen entweder des oberen (2) oder des unteren (3) Elements so angepasst sind, dass sie entweder aus dem oberen (2) oder dem unteren (3) Element vollständig ausgestanzt werden, um dem gegenüberliegenden Mitglied zu entsprechen.

9. Vorrichtung (1) nach Anspruch 1 oder 2, wobei das obere Element (2) eine Vorderwand aufweist, die so angepasst ist, dass sie mit den Gesichtsflächen der Schneidezähne, Eckzähne und Prämolaren des Oberkiefers in Kontakt steht, und denen Linguale Gaumenwände fehlen Anpassung an die palatinalen Oberflächen der oberen Zähne und eines unteren Elements (3) mit einer hinteren Wand, die so angepasst ist, dass sie mit den Lingualen Oberflächen der Schneidezähne, Eckzähne und Prämolaren des Unterkiefers in Kontakt steht, und fehlender Gesichtswände, um mit der Gesichtsbehandlung in Eingriff zu kommen Oberflächen der unteren Zähne.

10. Vorrichtung (1) nach einem der vorhergehenden Ansprüche, wobei das obere Element (2) und das untere Element (3) aus einem ungiftigen Kunststoffmaterial bestehen, das ein thermoplastisches Material ist, das geformt werden kann, um sich an ein anzupassen individuelles Gebiss durch mäßige Erwärmung.

11. Vorrichtung (1) nach Anspruch 1 oder 2, wobei das obere und das untere Element (2, 3) durch elastische Scharniere (4, 4') miteinander verbunden sind, die aus dem gleichen Material wie das obere (2) und das untere Element (3) bestehen.

12. Vorrichtung (1) nach Anspruch 1 oder 2, wobei sich die Scharniere (4, 4') in dem dorsalsten Teil der Vorrichtung (1) befinden, der aufgrund des Materials selbst eingebettet oder elastisch ist.

13. Vorrichtung (1) nach Anspruch 11 oder 12, bei der der dorsalste Teil der Scharniere (4, 4') am hintersten Teil konvex und am vordersten Teil der Scharniere (4, 4') konkav ist, wenn sie sich im Mund des Patienten befinden.

14. Vorrichtung (1) nach Anspruch 1 oder 2, wobei der Inkrementalmechanismus in den obersten mittleren vorderen Teil der Vorrichtung (1) eingebettet ist, der den Zähnen vom linken Prämolaren zum rechten Prämolaren am nächsten liegt, wenn er sich außerhalb des Mundes des Patienten befindet.

15. Kit, umfassend eine Vorrichtung (1) gemäß einem der vorhergehenden Ansprüche zusammen mit einem Temperaturindikator, der angepasst ist, um die Temperaturänderung auf eine erhöhte Temperatur anzuzeigen, bei der das Material der Vorrichtung (1) individuell geformt werden kann, und mit einem beheizbaren Metallstab kombiniert mit einem isolierenden Handstück für die Verwendung des Verriegelns des inkrementellen Gleitprozesses mittels Penetration beide Elemente am gewünschten nach vorne positionierten oberen Element (2) relativ zum unteren Element (3).

## Revendications

1. Dispositif anti-ronflement et anti-apnée obstructive du sommeil à avancement progressif de la mandibule (1) comprenant un élément supérieur (2) adapté pour engager la dentition maxillaire d'un humain et un élément inférieur (3) adapté pour engager la dentition mandibulaire d'un humain. , lesquels éléments (2, 3) sont réglables par incréments l'un par rapport à l'autre, **caractérisé en ce que** les éléments supérieur (2) et inférieur (3) sont articulés de manière élastique ensemble par des charnières postérieures (4, 4') qui sont intégrées aux éléments (2 , 3) et situé dans la partie la plus postérieure du dispositif, l'articulation étant adaptée pour permettre des mouvements physiologiques de la mâchoire inférieure dans le plan sagittal tout en conservant une position avant de la mâchoire inférieure par rapport à la mâchoire supérieure et en maintenant ainsi le passage des voies respiratoires dans le nasopharynx, l'oropharynx et l'hypopharynx sensiblement exempts d'occlusion.

2. Dispositif (1) selon la revendication 1 dans lequel l'élément inférieur (3) est réglable par incréments de l'élément supérieur (2) ou dans lequel l'élément supérieur (2) est réglable par incréments de l'élément inférieur (3).

3. Dispositif (1) selon la revendication 1 dans lequel le mécanisme incrémental est noyé dans les corps des éléments supérieur (2) et inférieur (3), dans le corps de l'élément inférieur (3), ou dans le corps de l'élément supérieur (2).

4. Un dispositif (1) selon l'une quelconque des revendications 1 à 3, dans lequel le mécanisme incrémental est composé de multiples structures "snap-on-snap-of" incrémentielles intégrées dans ou pouvant être attachés aux corps des éléments supérieurs (2) ou inférieurs (3) les deux en même temps.

5. Dispositif (1) selon la revendication 4, dans lequel les multiples structures "snap-on-snap-of" incrémentielles sont constituées par des parties mâles contre femelles de formes différentes telles que rondes, carrées, hexagonales, etc., conçues pour s'engager avec sa partie opposée correspondante de la forme structurelle femelle.

6. Dispositif (1) selon la revendication 5, dans lequel les multiples structures snap-on mâles incrémentales sont lobées avec des nombres variables de segments ou dans lequel les multiples structures snap-on femelles incrémentielles sont lobées avec des nombres variables de segments.

7. Dispositif (1) selon les revendications 1 ou 2, dans lequel le mécanisme incrémental est configuré pour être retiré de l'élément supérieur (2), de l'élément inférieur (3) et des charnières (4, 4'), renouvelé, restauré et refixé à l'élément supérieur (2), l'élément inférieur (3) et les charnières (4, 4') à nouveau.

8. Dispositif (1) selon les revendications 5 à 7, dans lequel les structures femelles de l'élément supérieur (2) ou inférieur (3) sont adaptées pour être complètement poinçonnées hors de l'élément supérieur (2) ou inférieur (3) correspondre avec le membre adverse.

9. Dispositif (1) selon les revendications 1 ou 2 dans lequel l'élément supérieur (2) a une paroi antérieure adaptée pour être en contact avec les surfaces faciales des incisives, canines et prémolaires de la mâchoire supérieure et est dépourvue de parois palatines linguales pour s'adaptent aux surfaces palatines des dents supérieures, et un élément inférieur (3) ayant une paroi postérieure adaptée pour être en contact avec les surfaces linguales des incisives, canines et prémolaires de la mâchoire inférieure et dépourvues de parois faciales pour s'engager avec le visage surfaces des dents inférieures.

10. Dispositif (1) selon l'une quelconque des revendications précédentes, dans lequel l'élément supérieur (2) et l'élément inférieur (3) sont constitués d'une matière plastique non toxique qui est une matière thermoplastique qui peut être façonnée pour s'adapter à une dentition individuelle par chauffage modéré.

11. Dispositif (1) selon les revendications 1 ou 2 dans lequel les éléments supérieur et inférieur (2, 3) sont intégrés l'un à l'autre par des charnières élastiques (4, 4') faites du même matériau que les éléments supérieur et inférieur (2, 3).

12. Dispositif (1) selon les revendications 1 ou 2 dans lequel les charnières (4, 4') sont situées dans la partie la plus dorsale du dispositif (1) en étant noyées ou élastiques du fait du matériau lui-même.

13. Dispositif (1) selon les revendications 11 ou 12 dans lequel la partie la plus dorsale des charnières (4, 4') est convexe à la partie la plus postérieure et concave à la partie la plus antérieure des charnières (4, 4') lorsqu'il est situé dans la bouche du patient.

14. Dispositif (1) selon les revendications 1 ou 2, dans lequel le mécanisme incrémental est intégré dans la partie la plus frontale médiane maxillaire du dispositif (1) la plus proche des dents de la prémolaire gauche à la prémolaire droite lorsqu'il est située dans la bouche du patient.

15. Kit comprenant un dispositif (1) selon l'une quelconque des revendications précédentes avec un indicateur de température adapté pour indiquer le changement de température à une température élevée à laquelle le matériau du dispositif (1) peut être façonné individuellement, et avec un tige métallique chauffante combinée à une pièce à main isolante pour l'utilisation de verrouillage du processus incrémental coulissant au moyen de la pénétration des deux éléments au niveau de l'élément supérieur positionné vers l'avant souhaité (2) par rapport à l'élément inférieur (3).
